# EUROPEAN PATENT APPLICATION

(11) **EP 4 483 894 A1**
(43) Date of publication of application: **01.01.2025**
(21) Application number: 23760093.7
(22) Date of filing: 24.02.2023
(51) Int. Cl.: A61K 39/00, A61P 35/02, A61P 37/04, C07K 7/06, C12N 5/078, A61K 38/08

(54) **PHARMACEUTICAL COMPOSITION FOR TREATMENT OR PREVENTION OF ADULT T-CELL LEUKEMIA**

(30) Priority: 25.02.2022 JP 2022027480
(71) Applicant: NEC Corporation, 108-8001 Tokyo (JP); The University of Tokyo, Bunkyo-ku, Tokyo 113-8654 (JP); University of The Ryukyus, Nakagami-gun, Okinawa 903-0213 (JP); KURUME UNIVERSITY, Kurume-shi Fukuoka 830-0011 (JP)
(72) Inventor: UCHIMARU, Kaoru, Tokyo 113-8654 (JP); NAKANO, Kazumi, Tokyo 113-8654 (JP); YOSHIHARA, Yoshiko, Tokyo 108-8001 (JP); FUKUSHIMA, Takuya, Nakagami-gun, Okinawa 903-0213 (JP); TANAKA, Yuetsu, Nakagami-gun, Okinawa 903-0213 (JP); KARUBE, Kennosuke, Nakagami-gun, Okinawa 903-0213 (JP); MASUZAKI, Hiroaki, Nakagami-gun, Okinawa 903-0213 (JP); OSHIMA, Koichi, Kurume-shi, Fukuoka 830-0011 (JP); MIYOSHI, Hiroaki, Kurume-shi, Fukuoka 830-0011 (JP)
(74) Representative: Betten & Resch
(86) International application number: PCT/JP2023/006691
(87) International publication number: WO 2023/163094

(57) **Abstract**

As a technique that can be used for cancer immunotherapy of adult T-cell leukemia (ATL), provided is a pharmaceutical composition for treatment or prevention of adult T-cell leukemia, the pharmaceutical composition containing a peptide consisting of an amino acid sequence of any one of SEQ ID NOs: 1 to 68.

## Description

### Incorporation by Reference

This application is based upon and claims the benefit of priority from Japanese patent application No.2022-027480, filed on February 25, 2022, the disclosure of which is incorporated herein in its entirety by reference.

### Technical Field

The present invention relates to a pharmaceutical composition for treatment or prevention of adult T-cell leukemia (ATL). More specifically, the present invention relates to a pharmaceutical composition for treatment or prevention of ATL, the pharmaceutical composition containing a peptide consisting of a specific amino acid sequence.

### Background Art

Development of cancer immunotherapy using a cancer antigen peptide has been advanced. For example, Patent Literature 1 discloses cancer immunotherapy using a peptide derived from glypican 3 (GPC3) which is a cancer antigen. It has been revealed that GPC3 is expressed in almost all hepatocellular carcinomas, while GPC3 is hardly expressed in normal liver, liver cirrhosis, and the like. It is also known that GPC3 is highly expressed not only in a hepatocellular carcinoma but also in melanoma, ovarian cancer, and the like.

A cancer cell elimination reaction by a cytotoxic T lymphocyte (CTL) is induced by specific recognition of a cancer antigen (HLA epitope) consisting of 8 to 11 amino acids presented in a major histocompatibility antigen (HLA) class I molecule on a surface of a cancer cell by a T cell antigen receptor (TCR) on the CTL. HLA molecules are roughly classified into a class I molecule (HLA-A type, B type, or C type) and a class II molecule (HLA-DP type, DQ type, or DR type).

Human T-cell leukemia virus type 1 (HTLV-1) is a retrovirus that primarily infects a CD4 positive T cell and causes adult T-cell leukemia (ATL).

ATL is a malignant hematologic tumor that infiltrates various organs of the whole body, and is classified into four disease types of acute type, lymphoma type, chronic type, and smoldering type. The acute type and the lymphoma type are also referred to as "aggressive ATL", and are hematopoietic malignancies with the worst prognosis. The chronic type and the smoldering type are also referred to as "indolent ATL", a majority which causes blast crisis during the course and has poor prognosis.

Therapies for ATL include multi-agent combination chemotherapy. However, even in the latest report, a median survival time of "aggressive ATL" is 13 months, which is not satisfactory. A patient with "indolent ATL" may survive for a long period of time even without treatment, and is often followed up without treatment until a condition deteriorates due to blast crisis or the like.

### Citation List

### Patent Literature

Patent Literature 1: WO 2016/143816 A

### Summary of Invention

### Technical Problem

A main object of the present disclosure is to provide a technique that can be used for ATL cancer immunotherapy.

### Solution to Problem

In order to solve the above problems, the present disclosure provides the following [1] to [15].
[1] A pharmaceutical composition for treatment or prevention of adult T-cell leukemia (ATL), the pharmaceutical composition containing a peptide consisting of an amino acid sequence of any one of SEQ ID NOs: 1 to 68.
[2] The pharmaceutical composition according to [1], in which the peptide binds to one or more types of major histocompatibility antigen (HLA) molecules.
[3] The pharmaceutical composition according to [2], in which the peptide consists of an amino acid sequence of any one of SEQ ID NOs: 1 to 47 and binds to two or more types of HLA molecules.
[4] The pharmaceutical composition according to [3], in which the peptide consists of an amino acid sequence of any one of SEQ ID NOs: 1 to 33 and binds to three or more types of HLA molecules.
[5] The pharmaceutical composition according to any one of [1] to [4], in which the peptide induces a cytotoxic T lymphocyte (CTL).
[6] The pharmaceutical composition according to [5], in which the peptide consists of an amino acid sequence of any one of SEQ ID NOs: 16,19,20,21,47, and 66.
[7] The pharmaceutical composition according to any one of [1] to [6], which is in a form of a vaccine.
[8] A peptide consisting of an amino acid sequence of any one of SEQ ID NOs: 1 to 68.
[9] The peptide according to [8], which binds to one or more types of HLA molecules.
[10] The peptide according to [9], which consists of an amino acid sequence of any one of SEQ ID NO: 1 to 47 and binds to two or more types of HLA molecules.
[11] The peptide according to [10], which consists of an amino acid sequence of any one of SEQ ID NO: 1 to 33 and binds to three or more types of HLA molecules.
[12] The peptide according to any one of [8] to [11], which induces CTL.
[13] The peptide according to [12], consisting of an amino acid sequence of any one of SEQ ID NOs: 16,19,20,21,47, and 66.
[14] A method for producing an antigen-presenting cell having cytotoxic T lymphocyte inducing activity, the method including a step of bringing the peptide according to any one of [8] to [13] into contact with the antigen-presenting cell in vitro.
[15] Use of the peptide according to any one of [8] to [13] for producing a pharmaceutical composition for treatment or prevention of ATL.

### Advantageous Effects of Invention

The present disclosure provides a technique available for cancer immunotherapy of ATL.

### Brief Description of Drawings

Fig. 1 illustrates a result of stimulating peripheral blood mononuclear cells (PBMC) of an ATL patient (sample 1) with a peptide and measuring an IFN-γ production amount.
Fig. 2 illustrates a result of stimulating PBMC of an ATL patient (sample 3) with a peptide and measuring an IFN-γ production amount.
Fig. 3 illustrates a result of stimulating PBMC of an ATL patient (sample 4) with a peptide and measuring an IFN-γ production amount.
Fig. 4 illustrates a result of stimulating PBMC of an ATL patient (sample 12) with a peptide and measuring an IFN-γ production amount.
Fig. 5 illustrates a result of stimulating PBMC of an ATL patient (sample 21) with a peptide and measuring an IFN-γ production amount.
Fig. 6 illustrates a result of stimulating PBMC of an ATL patient (sample 24) with a peptide and measuring an IFN-γ production amount.
Fig. 7 illustrates a result of stimulating PBMC of an ATL patient (sample 29) with a peptide and measuring an IFN-γ production amount.

### Description of Embodiments

### 1. Immunogenic peptide

A peptide according to the present disclosure consists of an amino acid sequence of any one of SEQ ID NOs: 1 to 68.

The peptide according to the present disclosure is derived from six proteins (Casp-8, c-Myb, Fas, Helios, Wnt5a, and gp46) in which an abnormality is found in an amino acid sequence or four proteins (c-Myb, CADM1, EVC1, EVC2, p19, and tax) in which high expression is observed in an adult T-cell leukemia (ATL) patient.

When the peptide according to the present disclosure is derived from a protein in which an abnormality is found in an amino acid sequence in an ATL patient, the amino acid sequence of the peptide according to the present disclosure may include the abnormal amino acid sequence of the protein. The abnormal amino acid sequence of the protein only needs to be a sequence different from a wild-type amino acid sequence, and may be, for example, an amino acid sequence including fusion by deletion of the whole or a part of an amino acid sequence of exon (Fusion); replacement of the whole or a part of an amino acid sequence of exon (Alternative usage of exon); insertion of an amino acid sequence derived from the whole or a part of intron (Inclusion); replacement, deletion, or insertion of one or more amino acid sequences of exon; or the like.

When the peptide according to the present disclosure is derived from a protein in which high expression is observed in an ATL patient, the amino acid sequence of the peptide according to the present disclosure may consist of a part of a wild-type amino acid sequence of the protein.

Details of each of the genes Casp-8, c-Myb, Fas, Helios, Wnt5a, CADM1, EVC1, EVC2, p19, tax, and gp46 will be described below.

**[Table 1]**

| | Gene | Data base | ID |
|---|---|---|---|
| Casp-8 | (caspase 8) | HUGO Gene Nomenclature Commmittee (HGNC) | 1509 |
| c-Myb | (MYB proto-oncogene, transcription factor) | NGNC | 7545 |
| Fas | (Fas cell surface death receptor) | NGNC | 11920 |
| Helios | (IKAROS family zinc finger 2) | NGNC | 13177 |
| Wint5a | (Wnt family member 5A) | NGNC | 12784 |
| CADM1 | (cell adhesion molecule 1) | NGNC | 5951 |
| EVC1 | (EvC ciliary complex subunit 1) | NGNC | 3497 |
| EVC2 | EvC ciliary complex subunit 2 | NGNC | 19747 |
| p19 | | National Center for Biotechnology Information (NCBI) | NP_955617 |
| tax | | NCBI | BAD95658 |
| gp46 | | NCBI | NP_057865 |

Based on an abnormal amino acid sequence or a wild-type amino acid sequence of each of the above-described genes, a sequence consisting of nine amino acid residues predicted to exhibit a high binding property to a major histocompatibility antigen (HLA) molecule by a hypothesis obtained using an active learning experimental method (JP 8-151396 A) was selected. A peptide consisting of the selected amino acid sequences was synthesized, and binding ability to an HLA molecule and a cytotoxic T lymphocyte (CTL) inducibility using interferon γ (IFN-γ) production as an index were evaluated in vitro.

An amino acid sequence of a peptide and binding ability to an HLA molecule are presented in Table 2. The binding ability to an HLA molecule is indicated by a dissociation constant Kd value (-logKd value).

In the Table, "> -3.0" indicates that a Kd value is larger than -3.0 and there is no significant binding ability.

In addition, for example, description of "> -3.37" as seen in a measured value of "allele A*02:06" of SEQ ID NO: 26 indicates that a Kd value was smaller than -3.0 and significant binding ability was confirmed, but a Kd value could not be deterministically measured and was a value between -3.37 and -3.0.

**[Table 2]**

| SEQ ID NO. | Amino acid sequence | Measured value of allele A*24:02 | Measured value of allele A*02:01 | Measured value of allele A*02:06 |
|---|---|---|---|---|
| 1 | LLLFSAAAL | -4.955161 | -6.055175 | -4.963929 |
| 2 | YVYDPPTTI | -6.23388 | -6.157512 | -5.552212 |
| 3 | YTYDPPTTI | -5.507897 | -5.474238 | -5.174326 |
| 4 | YLYDPPTTI | -6.159959 | -6.234504 | -5.868 |
| 5 | AQAPPTAQL | -5.752784 | -6.017318 | -5.848128 |
| 6 | ALAPPTAQL | -5.329069 | -6.576803 | -6.406524 |
| 7 | ALLAPAVLL | -5.962306 | -6.666211 | -5.845153 |
| 8 | KFLEAFHEV | -6.877614 | -5.930552 | -5.80648 |
| 9 | CLDEHQWQL | -5.865796 | -6.664691 | -6.324304 |
| 10 | ATLAPAVLL | -5.204216 | -5.555678 | -6.115734 |
| 11 | AALDDLRTL | -4.87668 | -5.053362 | -6.072933 |
| 12 | ALPKIRARV | -6.180589 | -5.660504 | -4.779625 |
| 13 | LMEEHGATL | -5.223294 | -4.917532 | -5.67827 |
| 14 | SLVLTWAAL | -5.311076 | -5.627463 | -6.242613 |
| 15 | VLLQQFQTA | -5.550844 | -4.713793 | -5.468897 |
| 16 | LLITRKSFI | -6.638063 | -5.131336 | -4.625881 |
| 17 | LMITRKSFI | -6.012118 | -5.203411 | -4.532591 |
| 18 | ALPAPHLTL | -6.733862 | -5.993502 | -5.518169 |
| 19 | VMSHHGEKL | -6.100718 | -5.07986 | -4.297741 |
| 20 | IALETPVWI | -5.482722 | -5.109722 | -5.518556 |
| 21 | IQYSSFHNL | -6.531752 | -5.28612 | -5.600099 |
| 22 | ALQFLIPRL | -4.96382 | -5.900906 | -5.83809 |
| 23 | LLNPNRQTI | -6.220197 | -5.240723 | -3.528096 |
| 24 | MLPQTPSHL | -7.144786 | -5.578598 | -3.962718 |
| 25 | ATAPPTAQL | -4.535162 | -4.995851 | -6.164923 |
| 26 | ALRPAPALL | -6.401676 | -6.111296 | > -3.37 |
| 27 | AVLAPAVLL | -4.805983 | -5.152921 | -5.153776 |
| 28 | CIDEHQWQL | -3.478245 | -4.852411 | -5.250628 |
| 29 | LIITRKSFI | -5.602002 | -4.107437 | -3.637789 |
| 30 | LVITRKSFI | -5.459937 | -4.010582 | -3.614017 |
| 31 | ALQPPCPNL | -5.306697 | -5.128578 | -4.636105 |
| 32 | YQLSPPITW | -5.763919 | -3.455134 | -5.749343 |
| 33 | TLTAWQHGL | -4.695985 | -5.953639 | -5.847836 |
| 34 | ISAYRTCWI | -4.999866 | > -3 | -3.137432 |
| 35 | FSNDFGQSL | > -3 | -3.900423 | -4.714286 |
| 36 | CMIVHQGTI | -5.53543 | > -3 | -4.372437 |
| 37 | SSFEFFEEA | > -3 | -6.49437 | -7.423289 |
| 38 | HMAKVIEFL | -6.03476 | -5.686323 | > -3 |
| 39 | CTDEHQWQL | >-3 | -5.042581 | -6.795019 |
| 40 | LIEEHGATL | > -3 | -4.49659 | -4.814942 |
| 41 | LLEEHGATL | > -3 | -5.601954 | -5.303772 |
| 42 | LTEEHGATL | > -3 | -4.085562 | -4.923626 |
| 43 | LVEEHGATL | > -3 | -4.179687 | -4.861812 |
| 44 | YFFTVIFSF | -6.774954 | > -3 | > -4.4 |
| 45 | LTITRKSFI | -4.82322 | > -3 | -3.789322 |
| 46 | LAITRKSFI | -5.118736 | > -3 | -3.040449 |
| 47 | EAIDGYITL | -5.53598 | > -3 | -4.722986 |
| 48 | TVLVPPRNL | -5.270565 | > -3 | > -3 |
| 49 | AYRTCWIFS | -5.418114 | > -3 | > -3 |
| 50 | AQISAYRTC | > -3 | > -3 | -4.085741 |
| 51 | GTHLDAGTV | > -3 | > -3 | -3.661209 |
| 52 | YPGWHSTTI | -5.660838 | > -3 | > -3 |
| 53 | ETLQFIDSM | > -3 | > -3 | -3.37066 |
| 54 | VLPPARHST | > -3 | -5.111948 | > -3 |
| 55 | SSLDPPKVL | -5.349805 | > -3 | > -3 |
| 56 | SLPFSPSQF | -6.998552 | > -3 | > -3 |
| 57 | FIDSDSSSW | -4.701162 | > -3 | > -3 |
| 58 | CDLSSFEFF | -4.095109 | > -3 | > -3 |
| 59 | ATGDCSSFI | > -3 | -4.080581 | > -3 |
| 60 | LPFSPSQFL | -3.348308 | > -3 | > -3 |
| 61 | LAEEHGATL | > -3 | > -3 | -4.495261 |
| 62 | SLLITRKSF | -5.84291 | > -3 | > -3 |
| 63 | LHITRKSFI | -4.757461 | > -3 | > -3 |
| 64 | HMTSSERPF | -4.164965 | > -3 | > -3 |
| 65 | GYITCERPF | -6.52078 | > -3 | > -3 |
| 66 | VFGRVMQID | -5.412266 | > -3 | > -3 |
| 67 | VMQIDGVQP | > -3 | > -3 | -3.410122 |
| 68 | GRVMQIDGV | -4.631654 | > -3 | > -3 |
| 69 | SLPNEKQTS | > -3 | > -3 | > -3 |
| 70 | ILSDHQQSQ | > -3 | > -3 | > -3 |
| 71 | STGESAQTS | > -3 | > -3 | > -3 |
| 72 | GSPDEFSND | > -3 | > -3 | > -3 |
| 73 | SPDEFSNDF | > -3 | > -3 | > -3 |
| 74 | GILSDHQQS | > -3 | > -3 | > -3 |
| 75 | THLDAGTVE | > -3 | > -3 | > -3 |
| 76 | LPPARHSTI | > -3 | > -3 | > -3 |
| 77 | ARHSTIPLV | > -3 | > -3 | > -3 |
| 78 | IFADVSSST | > -3 | > -3 | > -3 |
| 79 | LHEEHGATL | > -3 | > -3 | > -3 |
| 80 | GYITLGKPH | > -3 | > -3 | > -3 |
| 81 | ASPSHMTSM | > -3 | > -3 | > -3 |

Peptides of SEQ ID NOs: 1 to 4, 23,and 48 are derived from CADM1.

Peptides of SEQ ID NOs: 5, 6, 24, 25, 36, 37, 52 to 60, and 76 to 78 are derived from c-Myb.

Peptides of SEQ ID NOs: 7 to 10, 26 to 28, 38, and 39 are derived from EVC1.

Peptides of SEQ ID NOs: 11 to 15, 40 to 43, 61, and 79 are derived from EVC2.

Peptides of SEQ ID NOs: 16, 17, 29, 30, 44 to 46, 62, and 63 are derived from Fas.

Peptides of SEQ ID NOs: 18 and 31 are derived from gp46.

Peptides of SEQ ID NOs: 19, 478, 64, 65, 80, and 81 are derived from Helios.

A peptide of SEQ ID NO: 20 is derived from p19.

Peptides of SEQ ID NOs: 21, 22, 32, and 33 are derived from tax.

Peptides of SEQ ID NOs: 34, 35, 49 to 51, and 69 to 75 are derived from Casp-8.

Peptides of SEQ ID NOs: 66 to 68 are derived from Wnt5a.

The peptide according to the present disclosure binds to one or more types (subtypes) of HLA molecules. The peptides according to the present disclosure binds to preferably two or more, more preferably three or more types of HLA molecules.

Peptides of SEQ ID NOs: 1 to 33 exhibit a binding property to all of a product of an HLA-A*24:02 gene (HLA-A*24:02 molecule), a product of an HLA-A*02:01 gene (HLA-A*02:01 molecule), and a product of an HLA-A*02:06 gene (HLA-A*02:06 molecule).

Peptides of SEQ ID NOs: 34 to 47 exhibit a binding property to any two or more of an HLA-A*24:02 molecule, an HLA-A*02:01 molecule, and an HLA-A*02:06 molecule.

Peptides of SEQ ID NOs: 48 to 68 exhibit a binding property to any one of an HLA-A*24:02 molecule, an HLA-A*02:01 molecule, and an HLA-A*02:06 molecule.

HLA subtypes to which the peptide according to the present disclosure can bind are not limited to HLA-A*24:02, HLA-A*02:01, and HLA-A*02:06. However, since these HLA subtypes cover about 85% of Oriental people including Japanese and about 55% of Western people, it is considered that a multi-HLA peptide of the present invention has a wide patient coverage in immunotherapy and the like.

Furthermore, the peptide according to the present disclosure induces CTL. In particular, peptides of SEQ ID NOs: 16,19,20,21,47, and 66 exhibit CTL inducing activity in an ATL patient.

The peptide according to the present disclosure has an HLA binding property and immunogenicity. As used in the present specification, "immunogenicity" means being capable of inducing an immune reaction (immunity inducibility), for example, increasing cytotoxic T lymphocyte (CTL) inducing activity of an antigen-presenting cell, and further increasing cytotoxic activity of CTL against a cancer cell.

In addition, as used in the present specification, "CTL induction" means that in vitro or in vivo, the peptide according to the present disclosure is presented on a surface of an antigen-presenting cell to induce or proliferate CTL that specifically recognizes a certain antigen, to differentiate a naive T cell into an effector cell having ability (cytotoxic activity) to kill a target cell such as a cancer cell, and/or to increase cytotoxic activity of the CTL.

The CTL inducing activity can be measured by evaluating cytokine (for example, IFN-γ) production by CTL. For example, CTL inducing activity may be measured by evaluating an increase in cytokine-producing cells induced from precursor cells by antigen-presenting cells such as peripheral blood mononuclear cells stimulated with the peptide according to the present disclosure using a known high-sensitivity immunoassay such as an Enzyme-Linked ImmunoSpot (ELISPOT) method or an Enzyme-Linked ImmunoSorbent Assay (ELISA) method.

The cytotoxic activity of CTL can also be measured by a known method such as a ⁵¹Cr release method.

When the activity is significantly increased as compared with a control, for example, when the activity is increased by 5% or more, 10% or more, 20% or more, preferably 50% or more, it can be evaluated that immunity or CTL is induced.

The peptide according to the present disclosure has a high HLA binding property and high CTL inducibility, and is therefore strongly expected to be useful as a cancer vaccine. Applications of the peptide according to the present disclosure to various immunotherapies, particularly to a dendritic cell therapy are also expected. In addition, the peptide according to the present disclosure can bind to a plurality of HLA types. Therefore, the peptide according to the present disclosure can provide a cancer vaccine, a dendritic cell therapy, and the like that cover an extremely wide ATL patient group.

In the peptide according to the present disclosure, amino acid residues constituting amino acid sequences of SEQ ID NOs: 1 to 68 or a part thereof may be modified as long as the peptide retains immunogenicity. The amino acid sequences represented by SEQ ID NOs: 1 to 68 are intended to be amino acid sequences in a state of being presented on an antigen-presenting cell. However, when the peptide is directly administered into a body, the peptide may undergo a change such as digestion of a terminal of the peptide in a digestive organ or the like depending on an administration route. Therefore, the peptide according to the present disclosure may be present in a form of a precursor having one or more amino acid residues and the like added to an N-terminal and/or a C-terminal before being incorporated into an antigen-presenting cell such that the amino acid residues represented by SEQ ID NOs: 1 to 68 are retained when the peptide binds to a predetermined HLA class I molecule on the antigen-presenting cell.

In the peptide according to the present disclosure, one or several amino acid residues may be replaced, inserted, deleted, or added, and/or modified in such a manner as glycosylation, side chain oxidation, and/or phosphorylation as long as the peptide has desired immunogenicity.

In the present specification, "amino acid" is used in its broadest meaning, and includes an artificial amino acid variant and a derivative in addition to a natural amino acid. In the present specification, examples of the amino acid include: a natural proteinaceous L-amino acid; a D-amino acid; a chemically modified amino acid such as an amino acid variant or a derivative; a natural non-proteinaceous amino acid such as norleucine, β-alanine, or ornithine; and a chemically synthesized compound having properties known in the art, the properties being characteristics of the amino acid. Examples of the non-natural amino acid include an α-methylamino acid (such as α-methylalanine), a D-amino acid, a histidine-like amino acid (such as β-hydroxy-histidine, homohistidine, α-fluoromethyl-histidine, or α-methyl-histidine), an amino acid having an extra methylene in a side chain ("homo" amino acid), and an amino acid in which a carboxylic acid functional group amino acid in a side chain is replaced with a sulfonic acid group (such as cysteic acid).

Regarding replacement of an amino acid residue and the like, those skilled in the art can appropriately replace an amino acid residue constituting the peptide according to the present disclosure in consideration of regularity of a peptide sequence that exhibits a binding property to HLA (J. Immunol., 152: p3913, 1994; Immunogenetics, 41: p178, 1995; J. Immunol., 155: p4307, 1994).

More specifically, in a case of a peptide that binds to an HLA-A*24:02 molecule, a second position amino acid constituting the peptide may be replaced with tyrosine, phenylalanine, methionine, or tryptophan, and/or an amino acid at a C-terminal may be replaced with phenylalanine, leucine, isoleucine, tryptophan, or methionine.

In addition, in a case of a peptide that binds to an HLA-A*02:01 molecule, a second position amino acid may be replaced with leucine or methionine, and/or an amino acid at a C-terminal may be replaced with valine or leucine.

Furthermore, in a case of a peptide that binds to an HLA-A*02:06 molecule, a second position amino acid may be replaced with valine or glutamine, and/or an amino acid at a C-terminal may be replaced with valine or leucine.

Peptides of SEQ ID NOs: 3 and 4 derived from CADM1 are each obtained by introducing a second position amino acid replacement into a peptide of SEQ ID NO: 2.

Peptides of SEQ ID NOs: 6 and 25 derived from c-Myb are each obtained by introducing a second position amino acid replacement into a peptide of SEQ ID NO: 5.

Peptides of SEQ ID NOs: 10, 27, 28, and 39 derived from EVC1 are each obtained by introducing a second position amino acid replacement into each of peptides of SEQ ID NOs: 7 and 9.

Peptides of SEQ ID NOs: 40 to 43, 61, and 79 derived from EVC2 are each obtained by introducing a second position amino acid replacement into a peptide of SEQ ID NO: 13.

Peptides of SEQ ID NOs: 17, 29, 30, 45, 46, and 63 derived from Fas are each obtained by introducing a second position amino acid replacement into a peptide of SEQ ID NO: 16.

The peptide according to the present disclosure can be produced using a technique known to those skilled in the art. For example, the peptide according to the present disclosure may be artificially synthesized by a solid phase method such as an Fmoc method or a tBoc method or a liquid phase method. In addition, a desired peptide may be produced by expressing a polynucleotide encoding the peptide according to the present disclosure or a recombinant vector containing the polynucleotide. In addition, any one of the peptides thus obtained can be identified using a technique known to those skilled in the art. Any one of the peptides thus obtained can be identified using, for example, an Edman degradation method or a mass spectrometry method.

### 2. Pharmaceutical composition

A pharmaceutical composition for treatment or prevention of ATL according to the present disclosure contains, as an active component, for example, a peptide consisting of amino acid residues of SEQ ID NOs: 1 to 68.

The peptide according to the present disclosure induces CTL by being presented on an antigen-presenting cell, and this induced CTL injures a cancer cell. Therefore, the active component of the pharmaceutical composition according to the present disclosure is not limited to the peptide according to the present disclosure, and may be a component capable of directly or indirectly inducing CTL, for example, a polynucleotide encoding the peptide or a vector containing the polynucleotide, an antigen-presenting cell that presents a complex of the peptide and an HLA molecule on a surface or an exosome secreted from the antigen-presenting cell, or a combination thereof. Examples of the antigen-presenting cell to be used include a macrophage and a dendritic cell, and it is preferable to use a dendritic cell having high CTL inducibility.

Other components known to be used for cancer treatment, for example, a chemokine, a cytokine, a tumor necrosis factor, a chemotherapeutic agent, and the like may be contained in the pharmaceutical composition according to the present disclosure. A dose of the peptide may be, for example, about 1 to 10 mg per day when a patient is an adult. However, the dose varies depending on the age of a patient, the weight of the patient, an administration method, and the like, and is therefore appropriately determined by those skilled in the art.

Although not intended to be limited, the pharmaceutical composition according to the present disclosure is considered to be useful for killing a cancer cell and the like by the following mechanism of action. The pharmaceutical composition according to the present disclosure is administered to an ATL patient, whereby the peptide in the pharmaceutical composition is presented on a surface of an antigen-presenting cell in a state of being binding to an HLA molecule. When CTL recognizes such a peptide on the antigen-presenting cell, CTL is activated, proliferates, and circulates in the whole body. When CTL specific to the peptide enters a cancer tissue, the CTL recognizes the same peptide derived from a specific cancer antigen naturally binding to an HLA molecule on a surface of a cancer cell, and kills the cancer cell. This function can contribute to treatment of cancer.

The pharmaceutical composition according to the present disclosure can be used not only for treatment of cancer but also for prevention of cancer. For example, by administering the pharmaceutical composition according to the present disclosure to a healthy human body, CTL is induced and the induced CTL remains in the body. Therefore, when a cancer cell develops, the CTL can injure the cancer cell. Similarly, by administering the pharmaceutical composition according to the present disclosure to a human body after cancer treatment, recurrence of cancer may be prevented.

The pharmaceutical composition according to the present disclosure can be dissolved in a water-soluble solvent, formulated in a form of a pharmaceutically acceptable salt, and administered to a patient. Examples of such a form of a pharmaceutically acceptable salt include a form buffered at a physiological pH in a form of a physiologically acceptable water-soluble salt, for example, in a form of a salt of sodium, potassium, magnesium, or calcium. In addition to the water-soluble solvent, a water-insoluble solvent can also be used, and examples of such a water-insoluble solvent include an alcohol such as ethanol or propylene glycol.

In addition, a formulation containing the pharmaceutical composition of the present embodiment may contain agents for various purposes, and examples of such an agent include a preservative and a buffer. Examples of the preservative include sodium bisulfite, sodium bisulfate, sodium benzalkonium thiosulfate chloride, chlorobutanol, thimerosal, phenylmercuric acetate, phenylmercuric nitrate, methylparaben, polyvinyl alcohol, phenylethyl alcohol, ammonia, dithiothreitol, and betamercaptoethanol. Examples of the buffer include sodium carbonate, sodium borate, sodium phosphate, sodium acetate, and sodium bicarbonate. These agents can be present in an amount by which pH of the system can be maintained between 2 to 9, preferably 4 to 8.

A dosage form of the pharmaceutical composition according to the present disclosure is not particularly limited, but when the pharmaceutical composition is used as a form of a vaccine, examples of the dosage form include an injection (muscle, subcutaneous, or intradermal), an oral formulation, and a nasal formulation. When the pharmaceutical composition according to the present disclosure is in a form of a vaccine, the vaccine may be a mixed cocktail vaccine containing a plurality of types of active components. For example, such a vaccine may contain any two or more of the peptides of SEQ ID NO: 1 to 68 or a plurality of types of active components in combination with other active components.

In addition, the vaccine may be an inactive component-containing vaccine containing a component that is a component other than the pharmaceutical composition, is not active by itself, and has an effect of further enhancing the effect of the pharmaceutical composition as the vaccine. Examples of the inactive component include an adjuvant and a toxoid. Examples of the adjuvant include a precipitating type adjuvant such as aluminum hydroxide, aluminum phosphate, or calcium phosphate, and an oily type adjuvant such as Freund's complete adjuvant or Freund's incomplete adjuvant, but are not intended to be limited thereto.

When the pharmaceutical composition according to the present disclosure is present in a form of a vaccine, the pharmaceutical composition is preferably administered into a body by injection or infusion, for example, by intradermal, subcutaneous, intravenous, or intramuscular administration, or by transdermal inhalation or inhalation from a mucous membrane of a nose, a pharynx, or the like. A single dose of the pharmaceutical composition can be set between an amount by which cytotoxic T lymphocytes can be significantly induced and an amount by which a significant number of non-cancer cells are not injured.

The pharmaceutical composition according to the present disclosure is intended not only for administration to a human body but also for use outside the body. More specifically, the pharmaceutical composition according to the present disclosure may be used in order to stimulate an antigen-presenting cell in vitro or ex vivo to increase CTL inducing activity. For example, a case where the pharmaceutical composition according to the present disclosure is used for cancer dendritic cell therapy will be exemplified. The pharmaceutical composition according to the present disclosure can be administered to a patient by previously being brought into contact with an antigen-presenting cell derived from a patient in need of treatment or prevention of cancer, such as a dendritic cell, and then returning the antigen-presenting cell to the patient's body. The peptide contained in the pharmaceutical composition can be introduced into the antigen-presenting cell by, for example, a lipofection method or an injection method. When a polynucleotide encoding the peptide according to the present disclosure is used in such an application, the polynucleotide can be introduced into the antigen-presenting cell by a technique known in the art. For example, the antigen-presenting cell derived from the patient may be transformed in vitro with a polynucleotide of interest or a vector encoding the polynucleotide by a lipofection method, an electroporation method, a microinjection method, a cell fusion method, a DEAE dextran method, or a calcium phosphate method.

### 3. Method for producing antigen-presenting cell

A method for producing an antigen-presenting cell according to the present disclosure includes a step of bringing peptides consisting of amino acid sequences of SEQ ID NOs: 1 to 68 into contact with the antigen-presenting cell in vitro.

It is considered that a peptide used in the production method according to the present disclosure binds to an HLA class I molecule on a surface of an antigen-presenting cell and is presented to CTL as an antigen peptide, thereby inducing CTL activity of the antigen-presenting cell. Therefore, what is brought into contact with the antigen-presenting cell is not limited to the peptide according to the present disclosure, and may be a component capable of directly or indirectly inducing CTL, for example, a polynucleotide encoding the peptide or a vector containing the polynucleotide, an antigen-presenting cell that presents a complex of the peptide and an HLA molecule on a surface or an exosome secreted from the antigen-presenting cell, or a combination thereof. Examples of the antigen-presenting cell to be used include a macrophage and a dendritic cell, and it is preferable to use a dendritic cell having high CTL inducibility.

An antigen-presenting cell produced by the production method according to the present disclosure is intended to be used not only as an active component of the pharmaceutical composition or an immunity inducer but also for immunotherapy or the like. For example, a case where the antigen-presenting cell is used for cancer dendritic cell therapy will be exemplified. The antigen-presenting cell produced can be administered to a patient by previously being brought into contact with an antigen presenting cell derived from a patient in need of immune induction, such as a dendritic cell having low CTL inducibility, and then returning the antigen presenting cell to the patient's body. The peptide according to the present disclosure can be introduced into the antigen-presenting cell by, for example, transfection via liposome (lipofection method) or an injection method. When a polynucleotide encoding the peptide according to the present disclosure is used in such an application, the polynucleotide can be introduced into the antigen-presenting cell by a technique known in the art. For example, the antigen-presenting cell derived from the patient may be transformed in vitro with a polynucleotide of interest or a vector encoding the polynucleotide by a lipofection method, an electroporation method, a microinjection method, a cell fusion method, a DEAE dextran method, or a calcium phosphate method.

### Example

### 1. Extraction of amino acid sequence

Procedures of prediction, experiment, and evaluation in the present Example were performed based on an active learning experimental plan described in WO 2006/004182 A, and the following steps were repeated as a whole to construct a rule.

(1) One trial of a lower-level learning algorithm described later is performed. That is, a plurality of hypotheses are expressed from random resampling of accumulated data, and a point having the largest variance of a prediction value for a randomly expressed question candidate point (peptide) is selected as a question point to be tested.
(2) The peptide of the selected question point is produced by a synthesis and purification method described later, and actual binding ability is measured by an experiment described later and added to the accumulated data.

By performing such an active learning method, it was possible to reduce the number of binding experiments that are originally required to be performed on a peptide consisting of nine amino acid residues for all 500 billion (= 20⁹) or more candidate substances of an HLA binding peptide.

According to the rule as described above, the amino acid sequences represented by SEQ ID NOs: 1 to 81 were extracted. Specifically, an amino acid sequence having a predicted binding score of 3 or more in terms of a -logKd value with a product of an HLA-A*24:02 gene (HLA-A*24:02 molecule), a product of an HLA-A*02:01 gene (HLA-A*02:01 molecule), and a product of an HLA-A*02:06 gene (HLA-A*02:06 molecule) was selected.

**[Table 3]**

| SEQ ID NO | Amino acid sequence | Predicted value of allele A*24:02 | Predicted value of allele A*02:01 | Predicted value of allele A*02:06 |
|---|---|---|---|---|
| 1 | LLLFSAAAL | 5.3954 | 5.6122 | 5.4844 |
| 2 | YVYDPPTTI | 5.2483 | 5.3878 | 5.3878 |
| 3 | YTYDPPTTI | 5.2849 | 5.341 | 5.0547 |
| 4 | YLYDPPTTI | 5.7999 | 6.2767 | 5.4006 |
| 5 | AQAPPTAQL | 4.947 | 4.5297 | 4.9742 |
| 6 | ALAPPTAQL | 5.5137 | 6.3111 | 5.7006 |
| 7 | ALLAPAVLL | 5.4892 | 5.6076 | 5.3213 |
| 8 | KFLEAFHEV | 5.4475 | 5.2151 | 5.8664 |
| 9 | CLDEHQWQL | 5.0394 | 5.1216 | 4.8845 |
| 10 | ATLAPAVLL | 4.9621 | 5.56 | 5.7903 |
| 11 | AALDDLRTL | 5.5918 | 5.0362 | 5.0734 |
| 12 | ALPKIRARV | 5.3585 | 5.5682 | 4.6902 |
| 13 | LMEEHGATL | 5.3357 | 5.7236 | 4.8107 |
| 14 | SLVLTWAAL | 5.3297 | 5.357 | 5.0377 |
| 15 | VLLQQFQTA | 4.9267 | 5.265 | 5.2232 |
| 16 | LLITRKSFI | 4.9325 | 4.9786 | 4.865 |
| 17 | LMITRKSFI | 5.1761 | 4.903 | 4.771 |
| 18 | ALPAPHLTL | 6.1275 | 4.9379 | 4.701 |
| 19 | VMSHHGEKL | 5.1725 | 4.3364 | 4.1436 |
| 20 | IALETPVWI | 5.0997 | 5.3423 | 5.108 |
| 21 | IQYSSFHNL | 5.9032 | 4.6083 | 5.3579 |
| 22 | ALQFLIPRL | 5.1527 | 5.0659 | 5.1431 |
| 23 | LLNPNRQTI | 5.7314 | 5.0241 | 4.4348 |
| 24 | MLPQTPSHL | 5.5978 | 5.3224 | 5.0358 |
| 25 | ATAPPTAQL | 4.9892 | 5.2349 | 5.8204 |
| 26 | ALRPAPALL | 5.8917 | 5.1698 | 4.7738 |
| 27 | AVLAPAVLL | 5.1909 | 5.5813 | 5.5063 |
| 28 | CIDEHQWQL | 5.0194 | 5.3401 | 5.8291 |
| 29 | LIITRKSFI | 4.9699 | 4.1916 | 4.2501 |
| 30 | LVITRKSFI | 4.6453 | 4.4255 | 4.7705 |
| 31 | ALQPPCPNL | 5.2403 | 4.8818 | 5.1202 |
| 32 | YQLSPPITW | 5.2881 | 4.4009 | 5.1576 |
| 33 | TLTAWQHGL | 4.9203 | 4.8909 | 4.4191 |
| 34 | ISAYRTCWI | 3.8267 | 4.0678 | 3.9299 |
| 35 | FSNDFGQSL | 4.5854 | 4.3705 | 3.7956 |
| 36 | CMIVHQGTI | 5.1993 | 5.301 | 4.593 |
| 37 | SSFEFFEEA | 3.4109 | 4.9633 | 4.5643 |
| 38 | HMAKVIEFL | 6.0024 | 4.9312 | 4.5233 |
| 39 | CTDEHQWQL | 4.84 | 5.1202 | 6.1246 |
| 40 | LIEEHGATL | 4.6546 | 5.122 | 4.5639 |
| 41 | LLEEHGATL | 5.1337 | 5.6417 | 5.0018 |
| 42 | LTEEHGATL | 4.5876 | 4.7387 | 4.7278 |
| 43 | LVEEHGATL | 4.9208 | 5.1172 | 4.8716 |
| 44 | YFFTVIFSF | 6:2086 | 4.0936 | 4.1279 |
| 45 | LTITRKSFI | 4.847 | 3.6475 | 4.2049 |
| 46 | LAITRKSFI | 4.2534 | 4.4135 | 4.6998 |
| 47 | EAIDGYITL | 4.7563 | 3.8951 | 4.5883 |
| 48 | TVLVPPRNL | 5.0304 | 4.6162 | 4.2269 |
| 49 | AYRTCWIFS | 5.4627 | 3.5213 | 3.2048 |
| 50 | AQISAYRTC | 4.3869 | 3.7377 | 4.5628 |
| 51 | GTHLDAGTV | 3.5569 | 4.0198 | 4.9566 |
| 52 | YPGWHSTTI | 4.8158 | 4.7384 | 4.406 |
| 53 | ETLQFIDSM | 4.3549 | 3.4982 | 4.424 |
| 54 | VLPPARHST | 4.4968 | 4.2431 | 5.3975 |
| 55 | SSLDPPKVL | 4.5358 | 4.8509 | 4.4647 |
| 56 | SLPFSPSQF | 5.7771 | 5.2367 | 4.401 |
| 57 | FIDSDSSSW | 4.5959 | 3.721 | 4.2743 |
| 58 | CDLSSFEFF | 5.5317 | 3.6074 | 3.879 |
| 59 | ATGDCSSFI | 5.0873 | 4.3748 | 4.6378 |
| 60 | LPFSPSQFL | 4.3883 | 4.2067 | 4.3114 |
| 61 | LAEEHGATL | 4.4813 | 5.0977 | 4.6394 |
| 62 | SLLITRKSF | 5.2341 | 4.2079 | 4.7207 |
| 63 | LHITRKSFI | 4.5085 | 4.0391 | 4.3093 |
| 64 | HMTSSERPF | 5.5799 | 3.8544 | 3.558 |
| 65 | GYITCERPF | 6.4771 | 3.2797 | 3.1197 |
| 66 | VFGRVMQID | 4.4473 | 3.1074 | 3.5402 |
| 67 | VMQIDGVQP | 3.6744 | 4.6173 | 4.3009 |
| 68 | GRVMQIDGV | 3.0177 | 4.5084 | 4.1937 |
| 69 | SLPNEKQTS | 4.1512 | 4.4894 | 4.4993 |
| 70 | ILSDHQQSQ | 4.5813 | 4.1746 | 4.0446 |
| 71 | STGESAQTS | 3.6495 | 4.277 | 4.5435 |
| 72 | GSPDEFSND | 4.3578 | 3.7201 | 3.8972 |
| 73 | SPDEFSNDF | 4.3723 | 3.4784 | 3.6062 |
| 74 | GILSDHQQS | 4.0334 | 4.0314 | 4.5669 |
| 75 | THLDAGTVE | 3.8107 | 4.1005 | 3.7226 |
| 76 | LPPARHSTI | 4.9947 | 4.4871 | 4.3819 |
| 77 | ARHSTIPLV | 3.6597 | 4.5648 | 5.2325 |
| 78 | IFADVSSST | 4.9891 | 3.7176 | 3.9608 |
| 79 | LHEEHGATL | 4.7524 | 4.6916 | 4.3323 |
| 80 | GYITLGKPH | 4.6376 | 3.3894 | 3.2538 |
| 81 | ASPSHMTSM | 4.7099 | 3.7652 | 4.2798 |

### 2. Peptide synthesis and purification

Peptides having amino acid sequences of SEQ ID NOs: 1 to 81 were manually synthesized by a solid phase method of Merrifield using a Fmoc amino acid. After deprotection, reverse phase HPLC purification was performed using a C18 column to set a purity to 95% or more. Identification of a peptide and confirmation of a purity of the peptide were performed by MALDI-TOF mass spectrometry (AB SCIEX MALDI-TOF/TOF5800). Quantification of a peptide was performed by Micro BCA assay (Thermo Scientific) using BSA as a standard protein.

### 3. Experiment of binding of peptide to HLA-A molecule

### (1) HLA-A*24:02 molecule

Measurement of binding ability of a peptide to an HLA-A*24:02 molecule, which is a product of an HLA-A*24:02 gene, was performed using C1R-A24 cells expressing an HLA-A*24:02 molecule (Cells created by Professor Masafumi Takiguchi, Kumamoto University, was provided by Assistant Professor Masaki Yasukawa, Ehime University, with permission.).

First, the C1R-A24 cells were exposed to acidic conditions at pH 3.3 for 30 seconds to dissociate and remove an endogenous peptide originally binding to an HLA-A*24:02 molecule and light chain β2m commonly associated with an HLA class I molecule. After neutralization, purified β2m was added to the C1R-A24 cells, and the mixture was added to a peptide dilution series and incubated on ice for four hours. Staining was performed using a fluorescently-labeled monoclonal antibody 17A12 that recognizes an association (MHC-pep) of three members of an HLA-A*24:02 molecule, a peptide, and β2m which had been reassociated during this time.

Thereafter, the number of MHC-peps for each C1R-A24 cell (proportional to a fluorescence intensity of the fluorescent antibody) was quantitatively measured using a fluorescence cell analyzer FACScan (Becton-Dickinson). From an average fluorescence intensity per cell, a binding/dissociation constant Kd value between an HLA-A*24:02 molecule and a peptide was calculated by a method disclosed in an article (Udaka et al., Immunogenetics, 51, 816-828, 2000) by the present inventor.

### (2) HLA-A*02:01 molecule

Measurement of binding ability of a peptide to an HLA-A*02:01 molecule, which is a product of an HLA-A*02:01 gene, was performed using a cell line T2 (purchased from ATCC) expressing an HLA-A*02:01 molecule.

T2 cells and purified β2m were added to a serial dilution series of a peptide whose binding ability was to be measured, and then the mixture was incubated at 37°C for four hours. HLA-A*02:01 molecules whose expression level increased in a peptide concentration-dependent manner by this time point were stained using an association-type specific fluorescently labeled monoclonal antibody BB7.2.

Thereafter, the amount of fluorescence per cell was measured with a flow cytometer, and a dissociation constant Kd value was calculated by the method disclosed in the article (Udaka et al., Immunogenetics, 51, 816-828, 2000) by the present inventor.

### (3) HLA-A*02:06 molecule

Measurement of binding ability of a peptide to an HLA-A*02:06 molecule, which is a product of an HLA-A*02:06 gene, was performed using RA2.6 cells (a cell line newly created at Kochi University) obtained by introducing cDNA of the HLA-A*02:06 gene into RMAS, which is a mouse transporter associated with antigen processing (TAP)-deficient cell line.

First, the RA2.6 cells were cultured overnight at 26°C, a peptide dilution series was added to accumulation of the HLA-A*02:06 molecules to which no peptide was binding on a cell surface, and bonding was performed at 26°C for 60 minutes.

Thereafter, by performing culture at 35°C for four hours, an empty HLA-A*02:06 molecule to which no peptide was binding was denatured, and a steric structure was lost. A fluorescently labeled monoclonal antibody BB7.2 that specifically recognizes a peptide binding type HLA-A*02:06 molecule was added thereto, and the mixture was incubated on ice for 20 minutes to stain the cells.

Thereafter, the amount of fluorescence per cell was measured with a flow cytometer, and a dissociation constant Kd value was calculated by the method described in the literature (Udaka et al., Immunogenetics, 51, 816-828, 2000).

### (4) Results

The dissociation constant Kd values are presented in Table 4. When the Kd value (-logKd value) is smaller than -3, it can be determined that there is a binding property.

**[Table 4]**

| SEQ ID NO. | Amino acid sequence | Measured value of allele A*24:02 | Measured value of allele A*02:01 | Measured value of allele A*02:06 |
|---|---|---|---|---|
| 1 | LLLFSAAAL | -4.955161 | -6.055175 | -4.963929 |
| 2 | YVYDPPTTI | -6.23388 | -6.157512 | -5.552212 |
| 3 | YTYDPPTTI | -5.507897 | -5.474238 | -5.174326 |
| 4 | YLYDPPTTI | -6.159959 | -6.234504 | -5.868 |
| 5 | AQAPPTAQL | -5.752784 | -6.017318 | -5.848128 |
| 6 | ALAPPTAQL | -5.329069 | -6.576803 | -6.406524 |
| 7 | ALLAPAVLL | -5.962306 | -6.666211 | -5.845153 |
| 8 | KFLEAFHEV | -6.877614 | -5.930552 | -5.80648 |
| 9 | CLDEHQWQL | -5.865796 | -6.664691 | -6.324304 |
| 10 | ATLAPAVLL | -5.204216 | -5.555678 | -6.115734 |
| 11 | AALDDLRTL | -4.87668 | -5.053362 | -6.072933 |
| 12 | ALPKIRARV | -6.180589 | -5.660504 | -4.779625 |
| 13 | LMEEHGATL | -5.223294 | -4.917532 | -5.67827 |
| 14 | SLVLTWAAL | -5.311076 | -5.627463 | -6.242613 |
| 15 | VLLQQFQTA | -5.550844 | -4.713793 | -5.468897 |
| 16 | LLITRKSFI | -6.638063 | -5.131336 | -4.625881 |
| 17 | LMITRKSFI | -6.012118 | -5.203411 | -4.532591 |
| 18 | ALPAPHLTL | -6.733862 | -5.993502 | -5.518169 |
| 19 | VMSHHGEKL | -6.100718 | -5.07986 | -4.297741 |
| 20 | IALETPVWI | -5.482722 | -5.109722 | -5.518556 |
| 21 | IQYSSFHNL | -6.531752 | -5.28612 | -5.600099 |
| 22 | ALQFLIPRL | -4.96382 | -5.900906 | -5.83809 |
| 23 | LLNPNRQTI | -6.220197 | -5.240723 | -3.528096 |
| 24 | MLPQTPSHL | -7.144786 | -5.578598 | -3.962718 |
| 25 | ATAPPTAQL | -4.535162 | -4.995851 | -6.164923 |
| 26 | ALRPAPALL | -6.401676 | -6.111296 | > -3.37 |
| 27 | AVLAPAVLL | -4.805983 | -5.152921 | -5.153776 |
| 28 | CIDEHQWQL | -3.478245 | -4.852411 | -5.250628 |
| 29 | LIITRKSFI | -5.602002 | -4.107437 | -3.637789 |
| 30 | LVITRKSFI | -5.459937 | -4.010582 | -3.614017 |
| 31 | ALQPPCPNL | -5.306697 | -5.128578 | -4.636105 |
| 32 | YQLSPPITW | -5.763919 | -3.455134 | -5.749343 |
| 33 | TLTAWQHGL | -4.695985 | -5.953639 | -5.847836 |
| 34 | ISAYRTCWI | -4.999866 | > -3 | -3.137432 |
| 35 | FSNDFGQSL | > -3 | -3.900423 | -4.714286 |
| 36 | CMIVHQGTI | -5.53543 | > -3 | -4.372437 |
| 37 | SSFEFFEEA | > -3 | -6.49437 | -7.423289 |
| 38 | HMAKVIEFL | -6.03476 | -5.686323 | > -3 |
| 39 | CTDEHQWQL | > -3 | -5.042581 | -6.795019 |
| 40 | LIEEHGATL | > -3 | -4.49659 | -4.814942 |
| 41 | LLEEHGATL | > -3 | -5.601954 | -5.303772 |
| 42 | LTEEHGATL | > -3 | -4.085562 | -4.923626 |
| 43 | LVEEHGATL | > -3 | -4.179687 | -4.861812 |
| 44 | YFFTVIFSF | -6.774954 | > -3 | > -4.4 |
| 45 | LTITRKSFI | -4.82322 | > -3 | -3.789322 |
| 46 | LAITRKSFI | -5.118736 | > -3 | -3.040449 |
| 47 | EAIDGYITL | -5.53598 | > -3 | -4.722986 |
| 48 | TVLVPPRNL | -5.270565 | > -3 | > -3 |
| 49 | AYRTCWIFS | -5.418114 | > -3 | > -3 |
| 50 | AQISAYRTC | > -3 | > -3 | -4.085741 |
| 51 | GTHLDAGTV | > -3 | > -3 | -3.661209 |
| 52 | YPGWHSTTI | -5.660838 | > -3 | > -3 |
| 53 | ETLQFIDSM | > -3 | > -3 | -3.37066 |
| 54 | VLPPARHST | > -3 | -5.111948 | > -3 |
| 55 | SSLDPPKVL | -5.349805 | > -3 | > -3 |
| 56 | SLPFSPSQF | -6.998552 | > -3 | > -3 |
| 57 | FIDSDSSSW | -4.701162 | > -3 | > -3 |
| 58 | CDLSSFEFF | -4.095109 | > -3 | > -3 |
| 59 | ATGDCSSFI | > -3 | -4.080581 | > -3 |
| 60 | LPFSPSQFL | -3.348308 | > -3 | > -3 |
| 61 | LAEEHGATL | > -3 | > -3 | -4.495261 |
| 62 | SLLITRKSF | -5.84291 | > -3 | > -3 |
| 63 | LHITRKSFI | -4.757461 | > -3 | > -3 |
| 64 | HMTSSERPF | -4.164965 | > -3 | > -3 |
| 65 | GYITCERPF | -6.52078 | > -3 | > -3 |
| 66 | VFGRVMQID | -5.412266 | > -3 | > -3 |
| 67 | VMQIDGVQP | > -3 | > -3 | -3.410122 |
| 68 | GRVMQIDGV | -4.631654 | > -3 | > -3 |
| 69 | SLPNEKQTS | > -3 | > -3 | > -3 |
| 70 | ILSDHQQSQ | > -3 | > -3 | > -3 |
| 71 | STGESAQTS | > -3 | > -3 | > -3 |
| 72 | GSPDEFSND | > -3 | > -3 | > -3 |
| 73 | SPDEFSNDF | > -3 | > -3 | > -3 |
| 74 | GILSDHQQS | > -3 | > -3 | > -3 |
| 75 | THLDAGTVE | > -3 | > -3 | > -3 |
| 76 | LPPARHSTI | > -3 | > -3 | > -3 |
| 77 | ARHSTIPLV | > -3 | > -3 | > -3 |
| 78 | IFADVSSST | > -3 | > -3 | > -3 |
| 79 | LHEEHGATL | > -3 | > -3 | > -3 |
| 80 | GYITLGKPH | > -3 | > -3 | > -3 |
| 81 | ASPSHMTSM | > -3 | > -3 | > -3 |

Peptides of SEQ ID NOs: 1 to 33 exhibited a binding property to all of the HLA-A*24:02 molecule, the HLA-A*02:01 molecule, and the HLA-A*02:06 molecule.

Peptides of SEQ ID NOs: 34 to 47 exhibited a binding property to any two or more of the HLA-A*24:02 molecule, the HLA-A*02:01 molecule, and the HLA-A*02:06 molecule.

Peptides of SEQ ID NOs: 48 to 68 exhibited a binding property to any one of the HLA-A*24:02 molecule, the HLA-A*02:01 molecule, and the HLA-A*02:06 molecule.

Peptides of SEQ ID NOs: 69 to 81 exhibited no binding property to any one of the HLA-A*24:02 molecule, the HLA-A*02:01 molecule, and the HLA-A*02:06 molecule, contrary to expectation.

### 4. Peptide immune induction test

Peripheral blood mononuclear cells (PBMC) separated from an ATL patient were cultured and stimulated with a peptide, and an IFN-γ production amount was measured by ELISA.

A suspension of PBMC (2 × 10⁶ cell/ml) was seeded in a 96 well plate (100 to 200 µl/well), and cultured in a medium containing 5 ug/ml of a peptide (ASF-104, autologous serum 2%, IL-2 20U/ml) for two to three days.

The cells were washed. Thereafter, the cells were resuspended in the same medium, 5 ug/ml of the peptide was added thereto, and the cells were further cultured for three to five days. PBMC that had not been subjected to peptide stimulation was used as a control.

A culture supernatant was collected and subjected to measurement of an IFN-γ production amount by ELISA (Human IFN-γ ELISA MAX Deluxe Set, manufactured by BioLegend, Inc.).

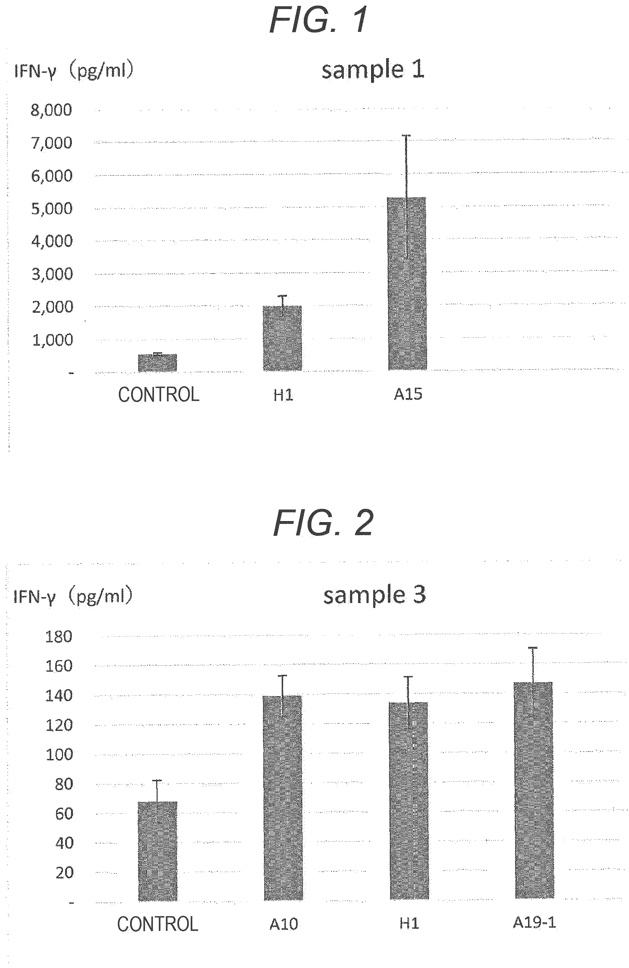

As results of a representative ELISA assay, results for seven patients are illustrated in Figs. 1 to 7.

Fig. 1 is a result for a patient with HLA-A*24:02 in one of alleles (sample 1).

Fig. 2 is a result for a patient with HLA-A*02:06 in one of alleles (sample 3).

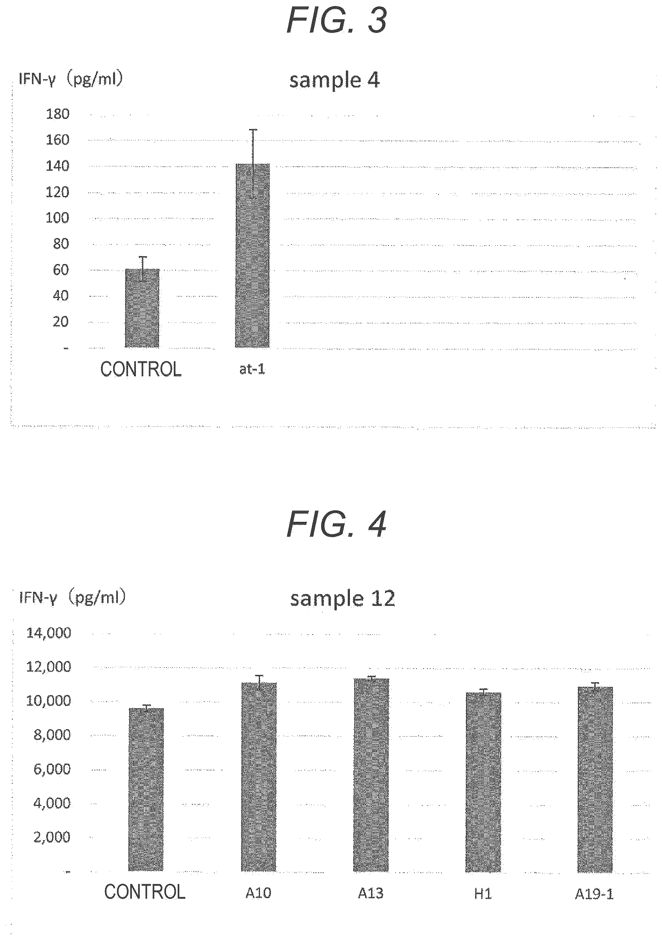

Fig. 3 is a result for a patient with HLA-A*02:06 in one of alleles (sample 4).

Fig. 4 is a result for a patient with HLA-A*02:01 in one of alleles (sample 12).

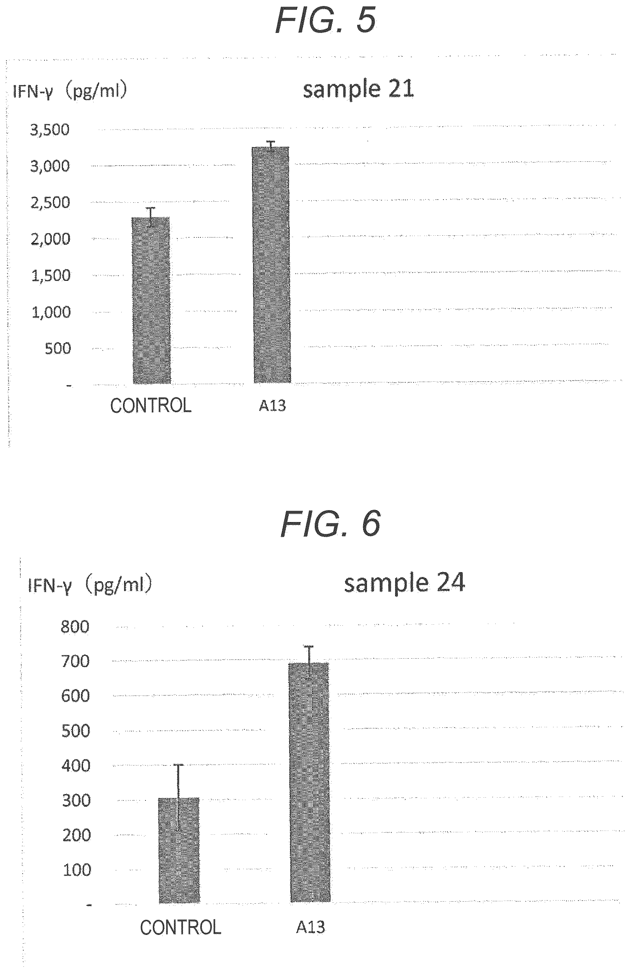

Fig. 5 is a result for a patient with HLA-A*02:01 in one of alleles (sample 21).

Fig. 6 is a result for a patient with HLA-A*24:02 in one of alleles (sample 24).

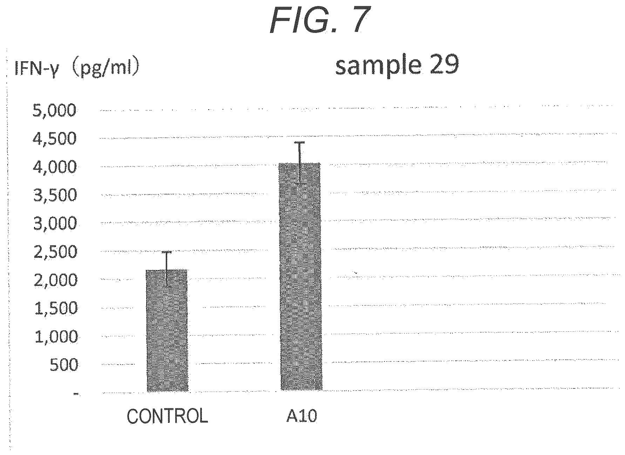

Fig. 7 is a result for a patient with HLA-A*24:02/*02:06 (sample 29).

In the drawings, "H1" means a peptide of SEQ ID NO: 47, "A15" means a peptide of SEQ ID NO: 66, "A10" means a peptide of SEQ ID NO: 16, "A19-1" means a peptide of SEQ ID NO: 20, "at-1" means a peptide of SEQ ID NO: 21, and "A13" means a peptide of SEQ ID NO: 19.

Significant production of IFN-γ was observed and immunity inducibility could be confirmed in peptides of SEQ ID NOs: 16, 19, 20, and 21 exhibiting a binding property to all of the HLA-A*24:02 molecule, the HLA-A*02:01 molecule, and the HLA-A*02:06 molecule.

In addition, significant production of IFN-γ was not observed in many of the peptides not exhibiting a binding property to all of the HLA-A*24:02 molecule, the HLA-A*02:01 molecule, and the HLA-A*02:06 molecule, but significant production of IFN-γ was observed and immunity inducibility could be confirmed in peptides of SEQ ID NOs: 47 and 66.

## Claims

1. A pharmaceutical composition for treatment or prevention of adult T-cell leukemia, the pharmaceutical composition comprising a peptide consisting of an amino acid sequence of any one of SEQ ID NOs: 1 to 68.

2. The pharmaceutical composition according to claim 1, wherein the peptide binds to one or more types of major histocompatibility antigen molecules.

3. The pharmaceutical composition according to claim 1 or 2, wherein the peptide induces a cytotoxic T lymphocyte.

4. The pharmaceutical composition according to any one of claims 1 to 3, which is in a form of a vaccine.

5. A peptide consisting of an amino acid sequence of any one of SEQ ID NOs: 1 to 68.

6. A method for producing an antigen-presenting cell having cytotoxic T lymphocyte inducing activity, the method comprising a step of bringing the peptide according to claim 5 into contact with the antigen-presenting cell in vitro.
